# EUROPEAN PATENT SPECIFICATION

(11) **EP 2 254 479 B1**
(45) Date of publication and mention of the grant of the patent: **17.09.2014**
(21) Application number: 08751485.7
(22) Date of filing: 26.02.2008
(51) Int. Cl.: A61B 8/06, A61B 5/0285

(54) **SYSTEM FOR DIAGNOSING MULTIPLE SCLEROSIS**
SYSTEM ZUR DIAGNOSE VON MULTIPLER SKLEROSE
SYSTÈME PERMETTANT DE DIAGNOSTIQUER LA SCLÉROSE EN PLAQUES

(43) Date of publication of application: 01.12.2010
(73) Proprietor: London Equitable Limited in its capacity as Trustee of the Think Tank Trust, Mayfair London W1J 7SX (GB)
(72) Inventor: ZAMBONI, Paolo, 44100 Ferrara (IT)
(74) Representative: Freyria Fava, Cristina
(86) International application number: PCT/IT2008/000129
(87) International publication number: WO 2009/107152

(56) References cited:
- WO-A-2006/102511
- WO-A-2007/088472
- US-B1- 6 390 979
- ALBAYRAK ET AL: "Extracranial carotid Doppler ultrasound evaluation of cerebral blood flow volume in COPD patients" RESPIRATORY MEDICINE, BAILLIERE TINDALL, LONDON, GB, vol. 100, no. 10, 1 October 2006 (2006-10-01), pages 1826-1833, XP005626621 ISSN: 0954-6111
- ZAMBONI, PAOLO; MENEGATTI, ERICA; BARTOLOMEI, ILARIA; GALEOTTI, ROBERTO; MALAGONI, ANNA M.; TACCONI, GIOVANNA; SALVI, FABRIZIO: "Intracranial Venous Haemodynamics in Multiple Sclerosis" CURRENT NEUROVASCULAR RESEARCH, vol. 4, no. 4, November 2007 (2007-11), pages 252-258, XP002501858

## Description

### Field of the invention

This disclosure concerns a system for determining obstructions to venous flow at an extracranial level.

This disclosure was devised by paying specific attention to its possible use in the diagnosis of multiple sclerosis.

### Description of the related art

Multiple sclerosis (MS) is an inflammatory demyelinating disease of the central nervous system of unknown origin, which is widely considered to be autoimmune in nature.

MS is currently considered as one of the most invalidating diseases with prevalence about 5-30/100.000 patients worldwide yearly and incidence about 1-3/100.000 patients worldwide yearly in medium risk zones.

Current therapy of MS includes the use of immunomodulatory drugs (e.g. interferon beta, glatiramer acetate), immunosuppressive drugs (e.g. mitoxantrone, azathioprine, cyclophosphamide), monoclonal antibodies (e.g. anti-alfa 4 integrin antibodies) and in some cases symptomatic treatments only.

Current diagnosis of MS includes clinical findings, magnetic resonance imaging of brain and spinal cord, laboratory screening for systemic autoimmune diseases, and cerebrospinal fluid analysis (see e.g. Polman et al. Ann. Neurol. 2005; 58:840-846).

More specifically, the present invention relates to a system for diagnosing multiple sclerosis according to the preamble of Claim 1, which is known, e.g. from ZAMBONI, PAOLO; MENEGATTI, ERICA; BARTOLOMEI, ILARIA; GALEOTTI, ROBERTO; MALAGONI, ANNA M.; TACCONI, GIOVANNA; SALVI, FABRIZIO: "Intracranial Venous Haemodynamics in Multiple Sclerosis" CURRENT NEUROVASCULAR RESEARCH, vol. 4, no. 4, November 2007 (2007-11), pages 252-258, XP002501858

### Object and summary of the invention

The need is therefore felt for improved solutions enabling as early as possible reliable detection of possible development of MS.

The object of this disclosure is providing such improved solutions.

According to the invention, the above object is achieved thanks to the subject matter recalled specifically in the ensuing claims, which are understood as forming an integral part of this disclosure.

An example of the invention provides an effective means to verify the presence and identify the nature of venous obstruction, and to determine whether this may represent a distinctive characteristic of MS.

An example of the invention provides a description of congenital and/or developmental malformations of the steno-obstructive type affecting the main pathways of extracranial cerebrospinal venous drainage and their association with clinically defined multiple sclerosis (CDMS).

An embodiment of the invention is a system for diagnosing multiple sclerosis based on the determination of the rate of reflux, increased indices of resistance in the cerebral veins for providing clinical data useful for diagnosing multiple sclerosis.

### Brief description of the drawings

The invention will now be described, by way of example only, with reference to the enclosed figures of drawing, wherein:
- Figure 1 shows the B-mode detection of venous obstruction;
- Figure 2 shows the flow velocity in the IJVs;
- Figure 3 shows the CSA and ΔCSA in MS and in controls;
- Figure 4 shows the location of the steno-obstructive malformations;
- Figure 5 shows the classification and distribution of the venous malformations;
- Figure 6 shows the selective venography in RR and SP case;
- Figure 7 shows the selective venography in PP cases; and
- Figure 8 is a schematic block diagram of a system as described herein;
- Figure 9 shows the selective venography and reflux along the azygous system.

### Detailed description of exemplary embodiments

Since the time of their earliest description (see e.g. Charcot, JM. Histology of 'sclerose en plaque' Gazette Hosp (Paris) 1868; 41: 559-566), it is known that plaques, which constitute the fundamental lesion in multiple sclerosis, have a vein running through their center. These veins are dilated and split the MS lesions longitudinally, as demonstrated by autopsic studies and magnetic resonance venography. Histological examination of the veins involved reveals unequivocally the presence of characteristic signs of impaired venous drainage, such as perivenous iron deposits and fibrin cuffs, particular to chronic venous disease (CVD).

MS and CVD have in common many other aspects that are involved in inflammatory processes and tissue degeneration, such as expression of adhesion molecules, matrix metalloproteinases hyperactivation (MMPs), macrophage and T lymphocyte infiltration and increased iron deposition.

The present disclosure involves the investigation of cerebral venous hemodynamics. By means of transcranial color-coded duplex sonography (TCCS) - a technique which demonstrated that physiological intracranial venous flow is monodirectional, and characterized by a slow velocity, and low resistance index - the inventors have noted (see e.g. Zamboni, P, Menegatti E, Bartolomei I, Galeotti R, Malagoni A.M, Tacconi G, Salvi F. Intracranial venous haemodynamics in multiple sclerosis. Curr Neurovasc Res 2007; 4) that, in the deep cerebral veins (DCVs) anatomically related to plaque disposition, the hemodynamic parameters are consistently altered, with a high frequency of inversion of the physiological flow direction. In addition, the inventors have also noted the indices of resistance were dramatically increased with respect to those of healthy control subjects, suggesting an obstruction to venous flow that was localized at an extracranial level.

An association is generally known to exist between extracranial venous obstructive malformations and clinically undefined disabling neurological disorders, or between vascular malformations different from those herein described and a disease clinically mimicking MS.

However, although the relationship between the venous system and MS lesions seems to be proved, the inventors have noted that to date no information on intracranial venous haemodynamics nor on extracranial venous obstructive malformations has been made available in MS patients.

Magnetic resonance imaging (MRI) has limitations when evaluating cerebral venous haemodynamics in relation to the physiological mechanisms impacting on the local flow patterns, especially during changes of posture and activation of the respiratory thoracic pump.

For this reason the inventors investigated the intracranial venous haemodynamics in MS using transcranial color-coded duplex sonography (TCCS) and extracranial EchoColor-Doppler (ECD). The inventors thus developed an original description of venous strictures affecting the main pathways of extracranial cerebrospinal venous drainage and their association with clinically defined multiple sclerosis (CDMS).

In that respect, the inventors also noted the associations between extracranial venous obstructive malformations and clinically undefined disabling neurological disorders, and between vascular malformations different from those herein described and a disease clinically mimicking MS without however revealing any association with CDMS.

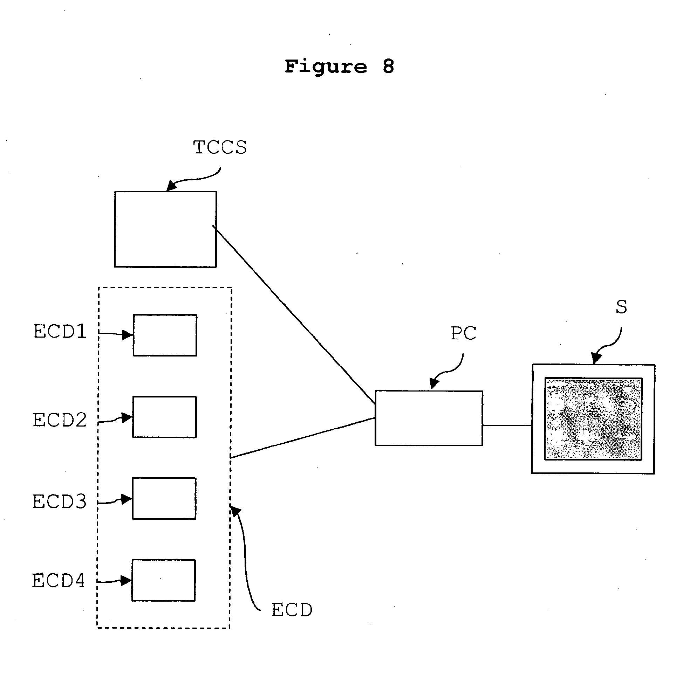

Figure 8 is a schematic block diagram of an exemplary embodiment of a system as described herein, i.e. a system for determining at least one index out of a blood reflux rate index and increased blood resistance index in cerebral veins in a patient. As detailed in the following, the presence of such indexes may be indicative of suspected patient's exposure to CDMS (Clinically Defined Multiple Sclerosis).

Specifically, the diagram of Figure 8 illustrates Transcranial Color-coded duplex Sonography apparatus (TCCS) and Extracranial EchoColor-Doppler apparatus (ECD).

Such apparatus is well known in the art and currently used in clinical practice. Exemplary of such equipment are e.g. the TCCS apparatus available under the trade designation of MYLAB25 or TECHNOS provided with high resolution probes of 2.5 MHz and the ECD apparatus available under the trade designation of MYLAB25 or TECHNOS provided with high resolution probes of 7.5-13 MHz from ESAOTE BIOMEDICA (Italy).

The TCCS and ECD apparatus in question are connected to a processing equipment such as e.g. a personal computer (PC) to process the detection signals produced by the TCCS and ECD apparatus that form a detection source set for the system herein.

Specifically, the TCCS apparatus comprises a first detection source adapted to detect (in manner known per se) a blood reflux in at least one of the deep middle cerebral veins. Similarly, the ECD apparatus comprises a second detection source adapted to detect at least one of:
- i) a blood reflux in at least one of the internal jugular and/or vertebral veins;
- ii) a stenosis in at least one of the internal jugular vein;
- iii) a lack of doppler detectable blood flow in at least one of the internal jugular and/or vertebral veins; and
- iv) a negative difference between the cross-sectional area of at least one of the internal jugular veins in the supine posture and in the erect posture of said patient.

To that end, the ECD apparatus may include (again in a manner known per se) separate modules ECD1 to ECD4.

As a function of the detection data provided by the detection sources (TCCS, ECD) the processing module PC is thus in a condition to sense the condition where the set of detection sources detect the subsistence of at least two of the entities sensed, namely:
- the blood reflux in at least one of the deep middle cerebral veins is detected by said first detection source (as detected, e.g. by the TCCS apparatus), and
- the blood reflux in at least one of the internal jugular and/or vertebral veins,
- the the stenosis in at least one of the internal jugular vein,
- the lack of doppler detectable blood flow in at least one of the internal jugular and/or vertebral veins, and
- the negative difference between the cross-sectional area of at least one of the internal jugular veins in the supine posture and in the erect posture of said patient (being the last four entities detected e.g. by the ECD apparatus).

When such a situation is sensed, the processing unit (PC) emits - for instance in the form of a visual message on an associated screen S - a corresponding advice signal.

In the exemplary embodiment to which Figure 8 refers, the detection source set of the system thus includes two separate detection sources.

The first detection source is comprised of the TCCS apparatus, adapted for detecting the presence of blood reflux in at least one of the deep middle cerebral veins.

The second detection source is comprised of the ECD apparatus, adapted for detecting (at least one of):
- blood reflux in at least one of the internal jugular and/or vertebral veins;
- a stenosis in at least one of the internal jugular vein;
- the lack of doppler detectable blood flow in at least one of the internal jugular and/or vertebral veins; and
- a negative difference between the cross-sectional area.

Those of skill in the art will otherwise appreciate that the one illustrated is just one exemplary embodiment of the system disclosed herein.

Examples of the system disclosed herein may include recourse to different detection sources (i.e. different types of apparatus providing the same or equivalent detection information), or a different partition of the detection actions (i.e. the set of detection sources being comprised of a single integrated detection system). Also, examples of the system disclosed herein may provide for off-line provision of the detection signals, i.e. detection of the various entities concerned being effected separately and the values detected stored in view of subsequent processing.

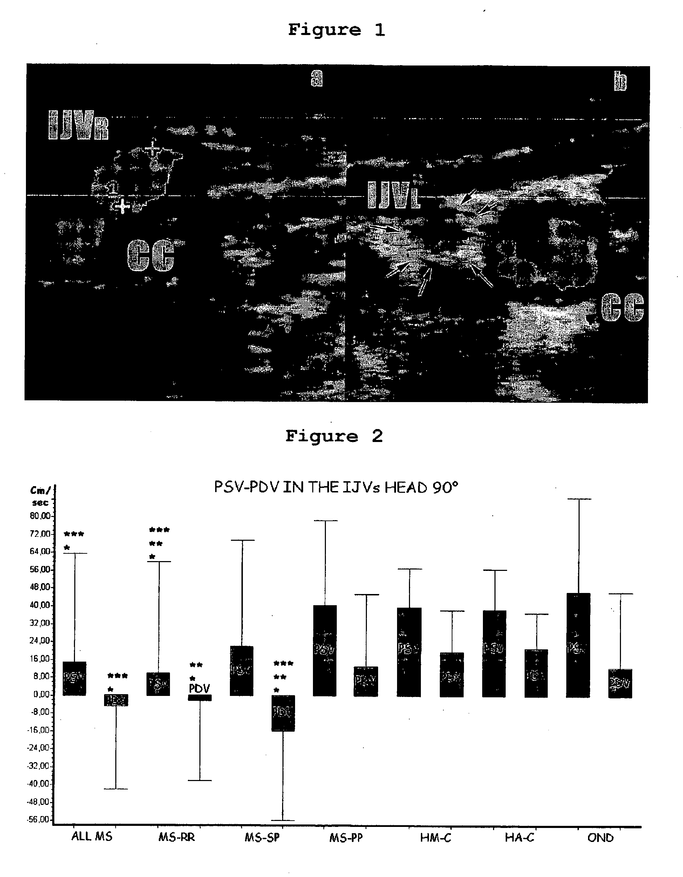

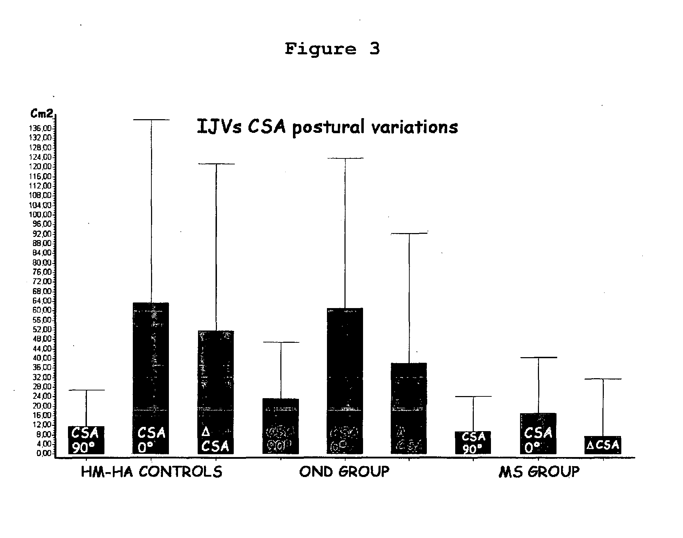

The malformations identified in the present disclosure are responsible for chronic extracranial venous outflow obstruction (CEVO), causing severe hemodynamic alterations, as documented in the first part of this disclosure. The physiological regulation of venous return is significantly altered by the presence of these malformed obstructions, with parameters that are significantly different from those of all control groups (Fig. 2, 3). CEVO must therefore be considered an exclusive characteristic of MS.

In fact, the hemodynamic parameters of venous obstruction adopted in the present study, confirmed in all cases by selective phlebography, were not present in the first control population, matched for age and gender with the MS population (60 healthy controls). For greater certainty, the inventors selected a second control population that was older than the average age of European MS patients, according to censuses in recent epidemiological studies (72 healthy aged subjects). Had the venous obstruction been present in this group, the inventors would not have been able to maintain that they have a role in the pathogenesis of MS, given that this control group had never had any neurological manifestations, nor other important diseases.

From this point of view, the fact of not having found impediments to cerebrospinal venous drainage even in the third control population, which was represented by other miscellaneous neurological diseases (45 patients) is of great interest. This finding would indicate that in no other disease of the nervous system can this chronically obstructed venous drainage be observed, not even in other pathologies that, like MS, present neurodegenerative, neuroimmunitary, and neurovascular aspects.

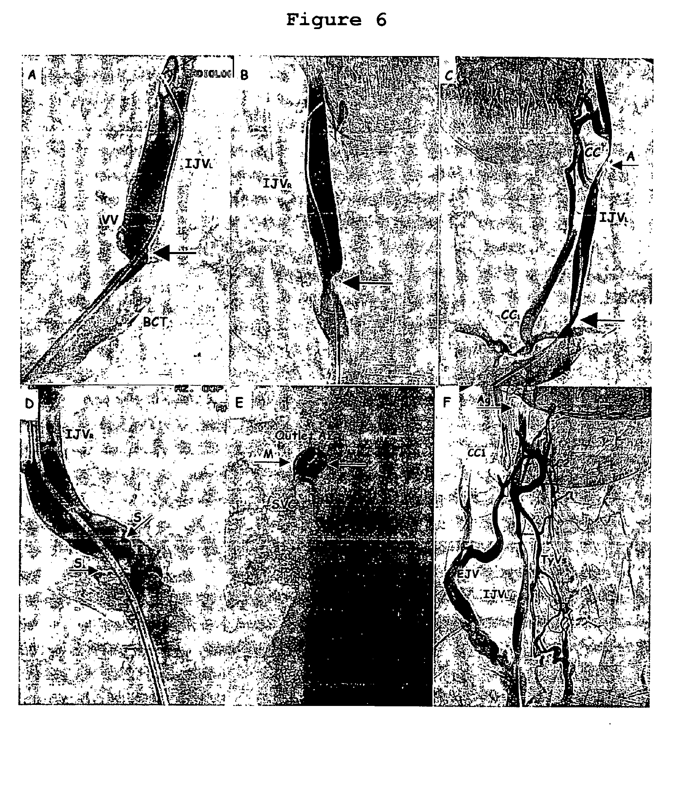

Some human diseases present similar malformations involving other venous segments. For example, membranous obstruction of the inferior vena cava upstream to the outlet of the suprahepatic veins is morphologically similar to the azygous membrane depicted in figure 6e; in the former position determines the appearance of Budd-Chiari syndrome. Venous septum, observed in the present study in the IJVs (Fig. 6d), has been also described in the inferior vena cava and in the iliac veins, where it brings about severe CVD in the lower limbs. Thus, outside the central nervous system, it is widely accepted that these venous malformations cause cellular degeneration and sclerosis: in the tissues drained by the affected veins the inventors respectively found hepatic cirrhosis, and lipodermatosclerosis with ulcer of the leg, as well as perivenous iron depositions.

Among the anomalies identified in the present disclosure, certainly the persistence of the reflux in any position of the head is the criterion most significantly associated with MS, with an Odds ratio (OR) of more than 6 (Table III). This reflux has a mechanism that differs from that caused by incompetence of the jugular valve. In the latter case, valvular .insufficiency tested with Valsalva can be related to a picture of transient global amnesia. In the present disclosure the reflux occurred naturally in any body position without the need to sollicit it by a forced movement. It is not the expression of valvular insufficiency but rather of the stenosing lesion that cannot be crossed with postural or respiratory mechanisms, becoming a long lasting reverse flow (Fig. 1). In other cases reflux is an expression of the opening of collateral circles that compensate for the reduced flow of the obstructed venous segment, in a direction that is opposite to the physiologic one.

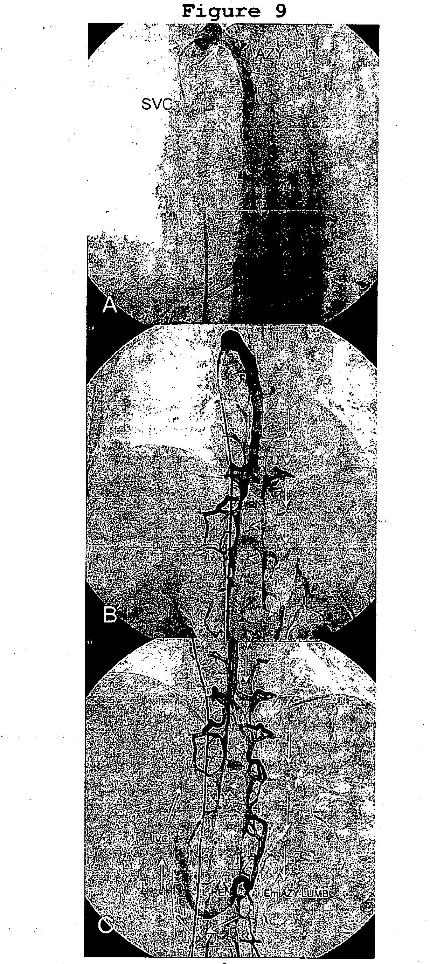

In the present disclosure stenosing lesions are defined as obstructions because the propelling mechanisms of cerebrospinal venous return (i.e., the muscular thoracic pump and postural variations) are functionally incapable of overcoming the stenoses. The example in Figure 9 is especially illuminating since it illustrates that a stenosis not morphologically closed in the proximal tract of the azygous vein in the supine position gives place to a reflux that is transmitted downward to the level of the lumbar plexuses. In order to be drained, this portion of countercurrent blood enters the intrarachidian plexuses, which become a substitute circle. Instead, the caval system gains in part, re-entering the inferior vena cava through the renal vein.

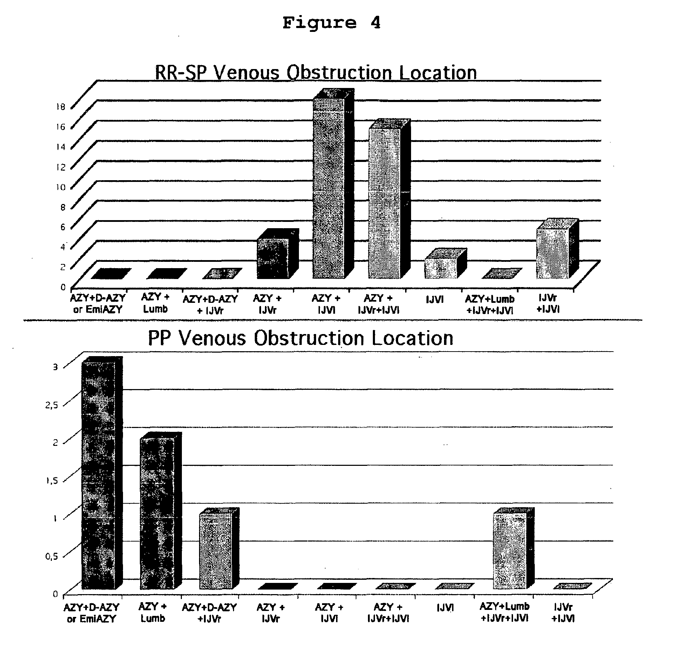

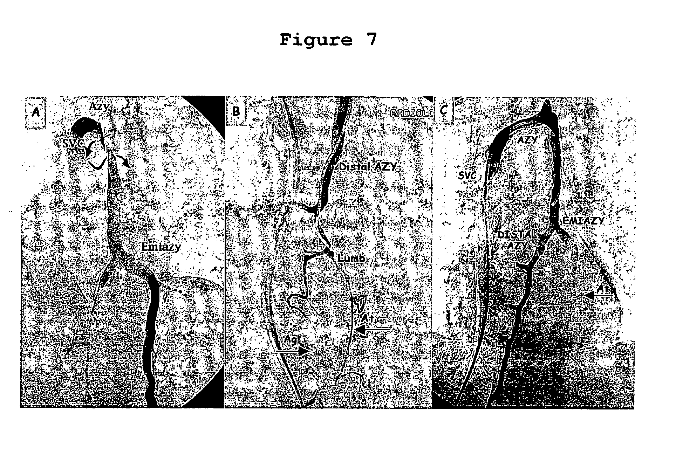

The present disclosure shows that within the MS group there exists a correlation between the topographies of the obstructive malformations and those of the MS lesions. In the relapsing-remitting (RR) and secondary progressive (SP) forms, with lesions mostly involving the brain and the cervical medulla, the venous obstructions are principally found in the jugular veins and in the proximal azygous vein (Fig. 4a, 6). In the PP form, with MRI documentation of thoraco-lumbar lesions, obstruction involves the veins that drain that particular territory, such as the distal azygous, hemiazygous, and lumbar veins (Fig. 4b, 7). The different topography of the MS lesions in the primary progressive (PP) form as compared to the RR and SP courses, and the further correspondence with the topography of the obstructed venous segments, suggests a pivotal role of venous drainage in the complex etiopathogenesis of this disease.

For example, the lack of drainage through the lumbar veins and/or the distal azygous discovered in PPMS would cause an ascending drainage through the intrarachidian veins. Such a circumstance is confirmed by the highest rate of intracranial reflux demonstrated in this subgroup (Table III), and could be related to the preferential distribution of plaques in the medulla. In contrast, the hampered drainage through the IJVs as well as in the proximal azygous found in the RR-SP patients seems to be related to the preferential onset and distribution of plaques in the brain.

The present disclosure also shows that within the MS group of patients pharmacologically treated with immunomodulating and/or immunosuppressive drugs (particularly with respect to RR-SP group) the obstructive malformations are not reduced as compared to the non-treated patients.

The venous strictures, demonstrated in patients who underwent phlebography, are responsible for CEVO, causing severe hemodynamic alterations. CEVO must therefore be considered an exclusive characteristic of MS, and dramatically increases the risk of MS by 36 fold (OR 36, 95% CI 22-57, p<0.0001).

The physiological regulation of venous return is significantly altered by the presence of these malformed obstructions, with parameters that are significantly different from those of all control groups, each one significantly increasing the risk of MS (Tab. III, Figs. 1 and 3).

### METHODS

### FIRST PHASE: NONINVASIVE SCREENING

### Patients and Controls

109 consecutive patients affected by clinically defined MS (CDMS), diagnosed according to the recommended criteria (as set forth in e.g. Polman, CH; Reingold, SC; Edan, G; Filippi, M; Hartung, H-P. Diagnostic Criteria for Multiple Sclerosis: 2005 Revisions to the "McDonald Criteria" Ann Neurol 2005; 58: 840-846) were admitted to the first part of the study. They were subdivided into 69 with a relapsing-remitting (RR) clinical course, 31 secondary progressive (SP), and 9 primary progressive (PP), attributing to each group a relative expanded disability disease score (EDDS).

These patients were compared with a blind design to 177 controls, subdivided into three groups: the first group included 60 healthy subjects matched for age and gender with MS patients (HM-C); the second control group included 72 healthy subjects older than the median age of the European MS population (HA-C), and the third group included 45 patients affected by other neurological diseases (OND) (Table I). The OND patients were subdivided into patients affected by neurodegenerative disorders (Parkinson's disease and amyotrophic lateral sclerosis-ALS), other neuroimmunitary disorders (OIND, including myasthenia gravis and multifocal motor neuropathy, MMN), and cerebro-vascular disease (ischemic stroke, transient ischemic attack-TIA) (Table II).

### Exclusion criteria

We excluded from the study those subjects having, or showing the potential for developing, a nervous system pathology of a venous refluxive and/or obstructive nature, including:
1. Chronic venous insufficiency of the lower limbs-CVI
2. History of venous thrombosis and\or post-thrombotic syndrome
3. Genetic thrombophilia
4. Congenital angiodysplasias
5. Congenital vascular malformations
6. Budd-Chiari syndrome
7. Behcet disease
8. Other Vasculitis

Patients and controls blindly underwent to a non-invasive study of cerebro-spinal venous return.

By applying the above stated exclusion criteria 109 CDMS patients entered the study; they were subdivided into 69 with a relapsing-remitting (RR) clinical course, 31 secondary progressive (SP), and 9 primary progressive (PP), attributing to each group a relative expanded disability disease score (EDSS). The control groups included 60 HM-C, 72 HA-C, and 45 OND patients (Table I).

**Table I.**

| | **All MS Patients (n=109)** | **Group HM-C (n=60)** | **Group HA-C (n=72)** | **Group MS-RR (n=69)** | **Group MS-SP (n=31)** | **Group: MS-PP (n=9)** |
|---|---|---|---|---|---|---|
| **AGE Median (yy) (25^{th}-75^{th} percentile)** | 40 (34-46) | 37 (28-49) | 58 (50.5-71.5) | 38 (30-43) | 44 (40-51) | 57 (46-60) |
| **Sex %M M/F** | 41% 45/64 | 46% 28/32 | 40% 29/43 | 40.5% 28/41 | 42% 13/18 | 44% 4/5 |
| **EDDS median (25^{th}-75^{th} percentile)** | 2 (1-4) | | | 1.5 (0.5-1.5) | 5.5 (4-7) | 5 (3.5-6.5) |
| **DISEASE DURATION(yy) median (25^{th}-75^{th} percentile)** | 6 (2-12) | | | 4 (2-7) | 13 (8-19) | 9 (4-14.5) |

**Table II.**

| | **Group OND (n=45)** | **Subgroup Neurodegenerative Disease (ALS;Parkinson's) (n=19)** | **Subgroup OIND (Myasthenia;MMN) (n=7)** | **Subgroup Cerebro-vascular Disease (Stroke; TIA) (n=19)** |
|---|---|---|---|---|
| **AGE median (25^{th}-75^{th} percentile)** | 60 (51-77) | 64 (52-76) | 50 (45-57) | 69 (53-78) |
| **Sex %M M/F** | 55.5% 25/20 | 47% 9/10 | 57% 4/3 | 63% 12/7 |

### Study of cerebrospinal venous drainage

Venous cerebrospinal return was examined, with the operators blinded of the diagnosis, with the subjects positioned on a tilt bed by combining the TCCS methodology for studying the deep cerebral veins (DCVs) with that of extracranial EchoColor-Doppler (ECD) for insonating the internal jugular veins (IJVs) and vertebral veins (VVs), both previously described. In particular we assessed the cerebrospinal venous return, focusing on the detection of five findings suggesting the presence of chronic extracranial venous outflow obstruction (CEVO). The detection of at least two findings was used for non invasive screening of highly suspected CEVO. Particularly we assessed:

### 1) Flow direction

Each measurement was preceded by a complete ECD high resolution B-mode exploration of the cervical vessels. We assessed the presence of reflux in the IJVs and VVs. According to a recent study on reflux time cut-off values, we considered reflux a flow reversal from its physiological direction for a duration > 0.88 sec. Flow direction was assessed during a short period of apnea following a normal exhalation, as previously reported, and not in a forced condition as Valsalva manouevre.

Furthermore, we assessed the eventual persistence of reflux with the head positioned at 0°, +15°, +30°, +45°, +90° in the four extracranial venous drainage pathways.

Therefore, TCCS investigation allows to detect, through the transtemporal window, the presence of reflux in at least one of the deep cerebral veins (DCVs)including the Galen, the basal, and the internal cerebral vein, eliciting venous flow by inviting the subject under examination to breathe, as previously reported.

### 2) Flow velocity.

We measured, at the level of the IJVs-VVs, respectively, in sitting and supine positions, the peak systolic velocity (PSV) and the peak diastolic velocity (PDV), both expressed in cm/sec. Measurement was derived from a 5 sec. recording of the Doppler spectrum analysis. PSV was the highest flow velocity recorded in systole, and PDV the highest flow velocity recorded in diastole during 5 sec of apnea, following normal expiration.

### 3) Postural control of cerebral venous outflow route

ECD clarified that physiologically the IJV is the predominant outflow pathway in the supine position, confirmed by an increased cross-sectional area (CSA) related to increased blood volume in that posture; redirection of venous flow to the VVs occurs in the upright position, with compliant reduction of the CSA of the IJV. Consequently we measured:
- The cross-sectional area (CSA) of both IJVs, in supine and sitting postures.
- The difference in CSA (delta CSA) obtained by subtracting the CSA measured in the supine from that in the erect position.

### ECD-TCCS criteria for venography

In Table III we report the list of the five criteria assessed through the ECD-TCCS protocol above described, and used for detection of significant cerebro-spinal venous flow disturbances and hampered venous outflow in patients and controls populations. Diagnosis of highly suspected CEVO required to fall-fill at least two of the five listed criteria. Highly suspected obstruction of cerebrospinal venous outflow pathways was taken as an indication to continue the study using selective venography in all identified subjects.

### SECOND PHASE: SELECTIVE VENOGRAPHY

### Patients and Treatments

Table IV shows the characteristics of the CDMS population under study. No significant differences were found in gender distribution among the three subgroups RR, SP, and PP, whereas age, EDSS, and disease duration were, of course, significantly higher in both progressive courses with respect to RR (p<0.05). HLA 2 DR15 genetic analysis was available in 44/51 patients, and CSF in 35/51, the latter mainly due to patient refusal. Finally, 48/51 patients fulfilled the revised MRI criteria of McDonald, whereas 3 patients, all belonging to the RR subgroup, did not. However, they fully satisfied either the clinical presentation or the additional CSF and MRI requirement. Table V lists the treatments administered in this cohort in the last three years. Due to lack of evidence, no treatments are listed for PP patients, and 14/44 patients RR/SP refused any pharmacological therapy.

**Table IV.**

| | **MS Patient population N°=51** | **MS RR N°=29** | **MS SP N°=15** | **MS PP N°=7** |
|---|---|---|---|---|
| **AGE median (25^{th}-75^{th} percentile)** | 39 (33-45) | 34 (29-39) | 44 (41-52) | 57 (42-60) |
| **Sex %M M/F** | 47% 24/27 | 48% 14/15 | 47% 7/8 | 42% 3/4 |
| **EDSS median (25^{th}-75^{th} percentile)** | 2 (1-4.5) | 1.5 (0.5-2) | 4.5 (3.5-6.5) | 5 (3.5-7.5) |
| **DISEASE DURATION (yy) median (25^{th}-75^{th} percentile)** | 6 (2-13) | 4 (1-7) | 13 (5-19) | 12 (2-15) |
| **HLA2 (DR15) Haplotype carriers (C) % C/no C** | 35% | 42% | 21% | 67% |
| | 15/28 | 10/14 | 3/11 | 2/3 |
| **CSF Oligoclonal Bands + % +/Tot** | 91% | 89% | 100% | 75% |
| | 32/35 | 16/18 | 13/13 | 3/4 |
| **Compliance with at least 3 of 4 MRI revised Mc Donald criteria % +/Tot** | 96% | 90% | 100% | 100% |
| | 49/51 | 26/29 | 15/15 | 7/7 |

**Table V.**

| **Drugs** | **N° MS Cases** |
|---|---|
| **Immunosuppressants** (Mitoxantrone, Cyclophosphamide, Azathioprine) | **18** |
| **Immunomodulators** (Interferon Beta, Glatiramer acetate,) | **21** |
| **Corticosteroids** (I.V. high doses Methylprednisolone) N° cycles in acute exacerbations | **88** |
| **Treatment refusal** | **14** |
| **PP cases** (no available effective treatment) | **7** |

### Statistical Analysis

Clinical and demographic characteristics are expressed as median and 25^{th}-75^{th} percentile. CSA in sitting and supine postures, delta CSA, PSV, and PDV are expressed as mean ± SD. Differences among groups were tested for significance with the ANOVA analysis of variance, with Bonferroni correction when p is <0.05.

The two-tailed Fisher's exact test followed by Odds ratio 95% Confidence Interval (CI) was used for determining the associated risk of MS in case of positive ultrasonographic criteria for CEVO, by comparing the whole MS group with a group including all controls.

The Odds ratio is a widely used statistic to compare the frequency of exposure to risk factors in epidemiological studies. Odds ratios compare the retrospective/posterior odds of exposure to a given risk factor in two groups of individuals. Odds ratios are interpreted with reference to a confidence interval (e.g. 95%). One can say that a given risk factor is a significant risk to a disease if the odds ratio is greater than one and the lower bound of the confidence interval does not go below one.

The two-tailed Fisher's exact test was also used for analyzing the different pattern of distribution of extra-cranial venous strictures in the RR/SP and PP groups, respectively, as well as for testing differences in the number of extracranial venous strictures between MS patients treated and not treated with drugs.

P-values up to 0.05 were considered statistically significant.

### RESULTS

### FIRST PHASE: NONINVASIVE SCREENING

### Patients and Controls

Tables I and II show clinical and demographic characteristics for the entire group of MS patients, and for the subgroups. Significant differences were found in the following:
- Age: SP vs RR, p<0.01. PP vs RR, p<0.01 (ANOVA).
- EDSS: SP vs RR, p<0.01. PP vs RR, p<0.01 (ANOVA).
- Disease duration: SP vs RR, p<0.01 (ANOVA).

### Study of cerebrospinal venous drainage

### 1) Flow direction

The persistence of reflux with the head positioned at 0°, +15°, +30°, +45°, +90° in at least one IJV and/or VV venous segments was never observed in any subject among the three control populations. By contrast, results were positive in 77/109 MS patients (70%), particularly in those with RR and SP courses, in 46/69 (66%) and in 28/31 (90%), respectively. Table III reports the sensitivity and specificity of such ECD-TCCS finding, in differentiating MS patients from controls.

In the PP course, reflux in the DCVs was more frequently observed as compared to RR-SP, 78% vs. 41%, respectively, and never in controls (p<0.0001).

**Table III.**

| **ECD-TCCS CRITERIA** | **MS-RR MS-SP (N; %)** | **MS-PP (N;%)** | **CONTROL POPULATIONS (N; %)** | **Odds Ratio All MS vs. All Controls (95% C.I.)** | **P** |
|---|---|---|---|---|---|
| **1. Reflux constantly present in an outflow pathway (IJV and\or W) with the head in any position (0°, +15°, +30°, +45°, +90°)** | 74/100 74% | 2/9 22% | 0/177 0% | 6.4 (4.7-8.7) | < 0.0001 |
| **2. Reflux propagated upward to the DCVs** | 41/100 41% | 7/9 78% | 0/177 0% | 3.9 (3.1-4.8) | < 0.0001 |
| **3. High resolution B-mode evidence of proximal IJV stenoses** | 27/100 27% | 3/9 33% | 0/177 0% | 3.2 (2.7-3.9) | < 0.0001 |
| **4. Flow not Doppler detectable in the IJVs and/or Ws despite numerous deep inspirations** | 35/100 35% | 3/9 33% | 0/177 0% | 3.5 (2.9-4.3) | < 0.0001 |
| **5. Negative ΔCSA in the IJV** | 58/100 58% | 3/9 33% | 21/177 12% | 3.2 (2.4-4.2) | < 0.0001 |

Finally, B-Mode analysis at high resolution allowed for the direct observation, in 30 MS patients, of the presence of closed stenosis in the proximal segment of an IJV, almost always the left (Fig. 1). Figure 1 shows the detection of venous obstruction in one MS patient by means of ECD (transversal access) with a probe 7,5 MHz; in A) the right cervical side has been observed, wherein common carotid artery (CC) and right internal jugular vein (IJVr) are shown. In B) the same patient has been evaluated with identification of stenosis of the left internal jugular vein (IJV1) due to annulus (black arrows encircling the stenosis), while the left common carotid artery (CC) appears normal.

### 2) Fl ow velocity

Figure 2 reports the highly significant differences in PSV and PDV values measured in the IJVs in the sitting position in the entire MS population and its subgroups, as compared to the three control populations. A further finding never seen in controls and recorded in 33% of RR-SP, and in 35% of PP cases, respectively, was the lack of flow velocity Doppler detectable in the IJVs and/or VVs despite numerous deep inspirations (Tab. III). It suggests a functional venous obstruction at the thoracic level.

Similarly, significant differences were also observed even with the head at 0°, and at the level of the VVs.

### 3) Postural control of cerebral venous outflow route

In Figure 3, the physiologic postural control of cerebral venous outflow route in the IJVs in both healthy control populations (HM-C, HA-C) is well apparent. CSA values in the sitting position are consistently lower than those assessed in the supine position, resulting in a rather big ΔCSA. The same correct physiologic response to a change in hydrostatic pressure condition was also demonstrated in the OND group, with no significant differences from the CSA assessed in the HM-C and HA-C groups (p<0.0001). In contrast, Figure 3 shows an overturning of this physiologic mechanism of postural regulation in the entire MS population. Redistribution of blood in the supine posture, in accordance with the principle of communicating vessels, seemed to be impeded in MS patients, and CSA at 0° was significantly lower in the MS patients than in the healthy controls, and even in the OND patients (p<0.0001). Consequently, the ΔCSA levels were significantly reduced in MS as compared to the three control groups, as shown in Figure 3. Finally, ΔCSA was negative in 56% of MS cases vs. 12% of the three control groups, as shown in Table III.

### ECD-TCCS criteria for venography

In Table III we report the list of the ECD-TCCS findings used for suspecting the presence of CEVO, and the relative distribution in RR-SP cases, PP cases, and in the controls. Each of the five findings demonstrated a noteworthy specificity and appreciable sensitivity in differentiating CDMS from the three control groups (Tab. III). As above reported, 70% of CDMS presented with reflux in any body posture in at least one of the four extracranial cerebral outflow routes vs. 0% of the control groups. However, in the 30% MS cases in which the ultrasound examination results had been negative for persistence of reflux in the IJVs/VVs in any body position, at least two of the other criteria were consistently positive. The control population never resulted positive for two criteria resulting in a 100% specificity of the five proposed criteria (Tab. III). Thus, by fulfilling the condition of two positive ECD-TCCS diagnostic criteria for hampered venous outflow, the proposed test was positive in 100% of the MS population, as opposed to 0% of all controls, both blindly investigated. Consequently, also the sensitivity by adopting the complex of the five criteria raised to 100%.

As a conclusion:
*1^{st} Criterion:* *Reflux in the IJVs and*/*or VVs with the head in any position.*
   The positiveness of this criterion increases dramatically the risk of MS by more than sixfold (OR 6.4, 95% CI 4.7-8.7, p<0.0001, Fisher's exact test).
*2^{nd} Criterion: Reflux in the DCVs*
   The presence of this finding increases significantly the risk of MS (OR 3.9, 95% CI 3.1-4.8, p< 0.0001, Fisher's exact test).
*3^{rd} Criterion: High resolution B-mode evidence of proximal IJV stenosis*
   The presence of closed stenosis in the proximal segment of an IJV increases the risk of MS (OR 3.2, 95% CI 2.7-3.9, p<0.0001, Fisher's exact test).
*4^{th}* *Criterion: Flow not Doppler detectable in the IJVs and*/*or VVs*
   This suggests a functional venous obstruction at the thoracic level and increases significantly the risk of MS, as indicated in Table III.
*5^{th} Criterion: Reverted postural control of the main cerebral venous outflow pathway*
   The ΔCSA levels significantly reduced in MS as compared to the three control groups results in a significantly increased risk of MS (Table III).

On the whole, the positiveness of two ECD-TCSS criteria of suspected CEVO dramatically increase the risk of CDMS (Odds Ratio= 77745, 95% Confidence Interval: 1530,1 to 3950364, p<0.0001) due to their exclusive detection in the MS group.

Stated otherwise, the embodiment described herein provides for the processing module PC to allot respective risk factor values to any of criteria discussed in the foregoing, i.e.:
- said blood reflux in at least one of the deep cerebral veins;
- said blood reflux in at least one of the internal jugular and/or vertebral veins,
- said stenosis in at least one of the internal jugular vein,
- said lack of Doppler detectable blood flow in at least one of the internal jugular and/or vertebral veins, and
- said negative difference between the cross-sectional area
as detected by said detection source set ECD, TCCS.

The processing module PC is thus in condition to derive and display for use by the practitioner a cumulative risk factor value, which is a function of the respective risk factor values.

While the cumulative risk factor value will tend to be higher if a higher number of risk criteria are met, the values of the risk factors allotted to each criterion met will also play a role in defining the cumulative risk factor of developing MS.

Table III above may thus be exemplary of an embodiment where the blood reflux in at least one of the internal jugular and/or vertebral veins is allotted a respective risk factor which is (substantially) higher - i.e. 6.4 - than any other respective risk factors allotted the other criteria (which may all fall in the range 3.2 to 3.9).

### SECOND PHASE: SELECTIVE VENOGRAPHY

### Patients and Treatments

Table IV shows the characteristics of the CDMS population under study, while table VI shows the results of the non invasive screening of this population (namely the number of positive TCCS-ECD criteria) performed according to the present invention.

**Table VI.**

| | **MS Patient population N°=51** | **MS RR N°=29** | **MS SP N°=15** | **MS PP N°=7** |
|---|---|---|---|---|
| **Number of positive TCCS-ECD criteria median (25^{th}-75^{th} percentile)** | 3 (2-3) | 3 (2-3) | 3 (3-3) | 2 (2-3) |

### Selective venography of the azygous and jugular venous system

Selective phlebography confirmed that the detection of at least 2/5 TCCS-ECD criteria of suspected CEVO, never measured in the control populations, were always related to a severe steno-obstruction, generally at the thoracic level, of the principal cerebrospinal venous segments. Interestingly, pattern of venous obstruction were significantly different located in RR-SP patients as compared to PP, and are given in Table VII, (p<0.0001, Fisher's exact test).

This result, when comparing ultrasonographic screening with invasive venography, confirmed the highly significant level of specificity of the former also in detecting CEVO (100%, P<0.0001).

**Table VII.**

| | **Multiple CEVO Confined to the Azygous territory** | **CEVO in Azygous and/or in the IJVs** | **P** |
|---|---|---|---|
| **RR-SP GROUP 44 PATIENTS** | 0 | 44 | < 0.0001 |
| **PP GROUP 7 PATIENTS** | 5 | 2 | < 0.0001 |

### Selective venography in RR and SP cases

This investigation showed the presence of obstructions in the proximal azygous vein and/or internal jugular veins in 100% of cases having RR and SP clinical courses (Table VII). Rarely (2/51, 4%) did these obstructions occur in only one IJV segment; almost always, two or three of the main venous outflow pathways were involved, thus severely compromising cerebrospinal venous drainage and demonstrating to be a multilevel pathology (Fig. 4a). Figure 4 shows the location in the cerebrospinal outflow veins of the steno-obstructive malformations in RR-SP (A), and PP cases (B), respectively. It should be emphasized that isolated obstruction is quite rare and that venous return is impaired by the combination of two or three stenoses. In addition, the topography of vein obstruction in the RR-SP groups (A) is different from that in the PP group (B) (IJV=internal jugular vein, left-1 and right-r; AZY=azygous vein; distal AZY=segment of the azygous vein below the emiazygousvein outlet; EMIAZY-Lumb= emiazygous vein and lumbar plexus). The number of extracranial venous wall stenoses did not differ significantly in patients treated with immunosuppressant/immunomodulator agents or in never-treated patients. In treated patients of the RR-SP class, the present inventors discovered up to 2 lesions in 36% of cases and > 2 lesions in 34%; in not treated patients, up to 2 lesions in 18% and > 2 lesions in 11% of cases (p=ns. Fisher's exact test).

Association between IJVs and proximal azygous obstruction was discovered in the vast majority (37/44, 84%) of cases (Fig. 4a), and azygous obstruction was mainly located at the junction with the superior vena cava and/or in its arch (Fig. 6E).

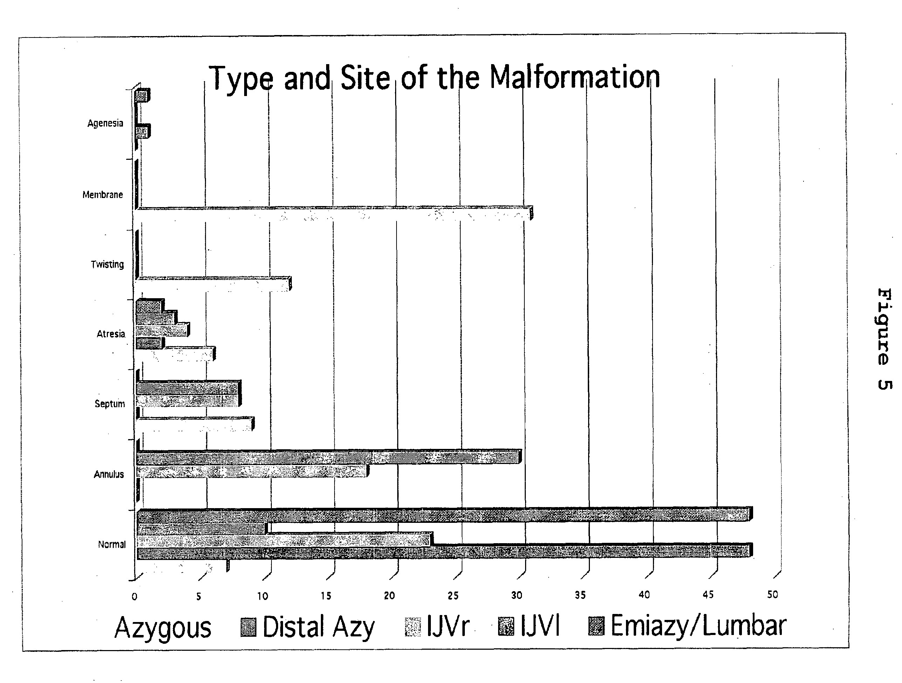

As to the morphology of these obstructions, selective venography revealed six principal venous malformations: annulus, agenesia, atresia, septum, membrane, and twisting. Figure 5 shows the relative distribution of the malformations found in the extracranial venous segments. Annulus was more likely to be found in the jugular system, whereas membranous obstruction seems to be typical of the azygous vein.

The panel in figure 6 provides the relative morphological details of the six malformation patterns, wherein in A) Annulus (arrow) at the level of the left internal jugular vein (IJV1) located immediately below the competent valve (VV) at the outlet with the brachiocephalic trunk (BCT) is shown. In B) Annulus (arrow) at the level of the right internal jugular vein (IJVr)and C) Combination of IJV1 atresia (arrow A) and annulus (arrow), compensated by two distinct collateral circles (CC) are shown. Unfortunately, the proximal CC re-enters at the level of the proximal annulus, thereby reducing its outflow contribution. In figure 6 D) Septum (arrow S) at the level of the IJVr, above the anonymous trunk (AnT), E) Membranous obstruction (arrow M) of the outlet of the azygous vein (AZY) in the superior vena cava (SVC), and F) Agenesia (Ag) of the distal segment of the right IJV, visible immediately above the tip of the catheter are shown. Supply flow through the condylar veins feed collateral circles, CC1 and CC2, in turn drained respectively into the external jugular vein (EJV), and into the thyroid veins (TyVs).

### Selective venography in PP cases

Also 7 PP cases with MRI-evident lesions, mostly at the level of the spinal cord, underwent venography. These patients presented, as a distinctive characteristic, a particular topography of the stenosed venous lesions that invariably involved the azygous vein, with a reduced association with the IJVs when compared to the RR and SP cases (Table VII) (Fig. 4b). In PP cases we identified a further association of proximal azygous stenoses with more distal venous obstructions, mainly at the level of the distal azygous, the hemiazygous vein, and often atresia/agenesia of the lumbar veins, not present in the RR and SP subjects (Fig. 4b, 5 and 7). This condition compromises drainage of the spinal cord at the thoracic-lumbar level, suggesting a strict relationship between the localization of venous obstructions and the clinical and MRI documentation of the site of the MS lesions.

In figure 7 venous lesions by means of selective venography in PP cases are shown. In A) Twisting of the proximal AZY (twisted arrow) with evident venous dilation below, involving also the emiazygous vein (Emiazy) is depicted. In B) Combination of atresia (At) and agenesia (Ag) involving the lumbar veins below the distal segment of the azygous vein (Distal Azy) and C) Atresia of the emiazygous vein (At) with normal azygous vein (AZY) from the outlet with the superior vena cava (SVC) to the distal segment (Distal Azy) are depicted.

Naturally, while the principle of the invention remains the same, the details of construction and the embodiments may widely vary with respect to what has been described and illustrated purely by way of example, without departing from the scope of the present invention as defined by the appended claims.

## Claims

1. A system for diagnosing multiple sclerosis by determining at least one index out of a blood reflux rate index and increased blood resistance index in cerebral veins in a patient, the system including a detection source set (TCCS, ECD) for detecting:
- a blood reflux in at least one of the deep cerebral veins,
**characterized in that** said detection source set is configured for further detecting at least one of:
- i) a blood reflux in at least one of the internal jugular and/or vertebral veins;
- ii) a stenosis in at least one of the internal jugular vein;
- iii) a lack of Doppler detectable blood flow in at least one of the internal jugular and/or vertebral veins; and
- iv) a negative difference between the cross-sectional area of at least one of the internal jugular veins in the supine posture and in the erect posture of said patient,
wherein the system includes a processing module (PC) operatively connected to said detection source set (TCCS, ECD) to sense the condition where at least two of:
- said blood reflux in at least one of the deep cerebral veins;
- said blood reflux in at least one of the internal jugular and/or vertebral veins,
- said stenosis in at least one of the internal jugular vein,
- said lack of Doppler detectable blood flow in at least one of the internal jugular and/or vertebral veins, and
- said negative difference between the cross-sectional area
are detected by said detection source set, wherein said processing module (PC) is configured for emitting (S) an advice signal indicative of suspected patient's exposure to multiple sclerosis in case said condition is sensed.

2. The system of claim 1, including:
- a first detection source (TCCS) of said blood reflux in at least one of the deep cerebral veins,
- a second detection source (ECD) of at least one of:
- i) said blood reflux in at least one of the internal jugular and/or vertebral veins;
- ii) said stenosis in at least one of the internal jugular vein;
- iii) said lack of Doppler detectable blood flow in at least one of the internal jugular and/or vertebral veins; and
- iv) said negative difference between the cross-sectional area of at least one of the internal jugular veins in the supine posture and in the erect posture of said patient, and
wherein said processing module is operatively connected to said first and second detection sources to sense said condition.

3. The system of claim 2, wherein said first detection source includes TransCranial Color-coded duplex Sonography (TCCS) apparatus.

4. The system of either of claims 2 or 3, wherein said second detection includes ExtraCranial EchoColor-Doppler (ECD) apparatus.

5. The system of any of the previous claims, wherein said processing module (PC) is configured to allot respective risk factor values to any of:
- said blood reflux in at least one of the deep cerebral veins;
- said blood reflux in at least one of the internal jugular and/or vertebral veins,
- said stenosis in at least one of the internal jugular vein,
- said lack of Doppler detectable blood flow in at least one of the internal jugular and/or vertebral veins, and
- said negative difference between the cross-sectional area
as detected by said detection source set, and to derive a cumulative risk factor value as a function of said respective risk factor values.

6. The system of claim 5, wherein said processing module (PC) is configured to allot to said blood reflux in at least one of the internal jugular and/or vertebral veins a respective risk factor value higher than any other respective risk factor value allotted by said processing module (PC).

## Patentansprüche

1. System zum Diagnostizieren von multipler Sklerose durch Bestimmen wenigstens eines Index' aus einem Blutrücklaufraten-Index und einem erhöhten Blutwiderstands-Index in Zerebralvenen eines Patienten, wobei das System eine Erfassungsquellengruppe (TCCS, ECD) umfasst um zu erfassen:
- einen Blutrücklauf in wenigstens einer der tiefen Zerebralvenen,
**dadurch gekennzeichnet, dass** die Erfassungsquellengruppe dazu eingerichtet ist zu erfassen:
- i) einen Blutrücklauf in wenigstens einer der inneren Hals- und/oder der Wirbelsäulenvenen und/oder
- ii) eine Stenose in wenigstens einer der inneren Halsvenen und/oder
- iii) einen Mangel des durch den Dopplereffekt erfassbaren Blutstroms in wenigstens einer der inneren Hals- und/oder der Wirbelsäulenvenen; und/oder
- iv) eine negative Differenz zwischen der Querschnittsfläche wenigstens einer der inneren Halsvenen in der Rückenschräglage und in der aufrechten Lage des Patienten,
wobei das System ein Verarbeitungsmodul (PC) umfasst, das wirkungsmäßig mit der Erfassungsquellengruppe (TCCS, ECD) verbunden ist, um den Zustand zu erfassen, in dem wenigstens zwei von:
- Blutrücklauf in wenigstens einer der tiefen Zerebralvenen;
- Blutrücklauf in wenigstens einer der inneren Hals- und/oder Wirbelsäulenvenen,
- Stenose in wenigstens einer der inneren Halsvenen,
- Mangel des durch den Dopplereffekt erfassbaren Blutstroms in wenigstens einer der inneren Hals- und/oder der Wirbelsäulenvenen und
- negativer Differenz zwischen der Querschnittsfläche
durch die Erfassungsquellengruppe erfasst werden, wobei das Verarbeitungsmodul (PC) dazu eingerichtet ist, ein Hinweissignal auszugeben (S), das für die angenommene Gefährdung des Patienten für multiple Sklerose für den Fall kennzeichnend ist, dass dieser Zustand erfasst wird.

2. System nach Anspruch 1, umfassend:
- eine erste Erfassungsquelle (TCCS) des Blutrücklaufes in wenigstens einer der tiefen Zerebralvenen,
- eine zweite Erfassungsquelle (ECD) für:
- i) den Blutrücklauf in wenigstens einer der inneren Hals- und/oder der Wirbelsäulenvenen und/oder
- ii) die Stenose in wenigstens einer der inneren Halsvenen und/oder
- iii) den Mangel des durch den Dopplereffekt erfassbaren Blutstroms in wenigstens einer der inneren Hals- und/oder der Wirbelsäulenvenen; und/oder
- iv) die negative Differenz zwischen der Querschnittsfläche wenigstens einer der inneren Halsvenen in der Rückenschräglage und in der aufrechten Lage des Patienten,
wobei das Verarbeitungsmodul (PC) wirkungsmäßig mit der ersten und der zweiten Erfassungsquelle verbunden ist, um den Zustand zu erfassen.

3. System nach Anspruch 2, bei dem die erste Erfassungsquelle eine TCCS-Vorrichtung (TCCS - TransCranial Color-coded duplex Sonography) umfasst.

4. System nach einem der Ansprüche 2 oder 3, bei dem die zweite Erfassung eine ECD-Vorrichtung (ECD - ExtraCranial EchoColor-Doppler) umfasst.

5. System nach einem der vorherigen Ansprüche, bei dem das Verarbeitungsmodul (PC) dazu eingerichtet ist, entsprechende Risikofaktorwerte
- dem Blutrücklauf in wenigstens einer der tiefen Zerebralvenen oder
- dem Blutrücklauf in wenigstens einer der inneren Hals- oder Wirbelsäulenvenen und/oder
- der Stenose in wenigstens einer der inneren Halsvenen oder
- dem Mangel des durch den Dopplereffekt erfassbaren Blutstroms in wenigstens einer der inneren Hals- und/oder der Wirbelsäulenvenen oder
- der negativen Differenz zwischen der Querschnittsfläche zuzuordnen,
wie sie durch die Erfassungsquellengruppe erfasst werden,
und einen kumulativen Risikofaktorwert in Abhängigkeit der entsprechenden Risikofaktorwerte abzuleiten.

6. System nach Anspruch 5, bei dem das Verarbeitungsmodul (PC) dazu eingerichtet ist, dem Blutrücklauf in wenigstens einer der inneren Hals- und/oder Wirbelsäulenvenen einen entsprechenden Risikofaktorwert zuzuordnen, der größer ist als ein beliebiger anderer entsprechender Risikofaktorwert, der von dem Verarbeitungsmodul (PC) zugeordnet wird.

## Revendications

1. Système de diagnostic de la sclérose en plaques par détermination d'au moins un indice parmi un indice de taux de reflux sanguin et un indice de résistance sanguine accrue dans les veines cérébrales d'un patient, le système comprenant un ensemble de sources de détection (TCCS, ECD) pour détecter :
- un reflux sanguin dans au moins l'une des veines cérébrales profondes, **caractérisé en ce que** ledit ensemble source de détection est configuré pour en outre détecter au moins l'un :
- i) d'un reflux sanguin dans au moins l'une des veines jugulaires et/ou vertébrales internes ;
- ii) d'une sténose dans au moins l'une des veines jugulaires internes ;
- iii) d'une absence de flux sanguin détectable au Doppler dans au moins l'une des veines jugulaires et/ou vertébrales internes ; et
- iv) d'un écart négatif entre la section transversale d'au moins l'une des veines jugulaires internes lorsque ledit patient se trouve en position allongée et en position debout,
dans lequel le système comprend un module de traitement (PC) fonctionnellement raccordé au dit ensemble de sources de détection (TCCS, ECD) pour détecter l'affection dans laquelle au moins deux :
- dudit reflux sanguin dans au moins l'une des veines cérébrales profondes,
- dudit reflux sanguin dans au moins l'une des veines jugulaires et/ou vertébrales internes,
- de ladite sténose dans au moins l'une des veines jugulaires internes ;
- de ladite absence de flux sanguin détectable au Doppler dans au moins l'une des veines jugulaires et/ou vertébrales internes, et
- dudit écart négatif entre les sections transversales,
sont détectés par ledit ensemble de sources de détection, dans lequel ledit module de traitement (PC) est configuré pour émettre (S) un signal d'information indiquant l'exposition à la sclérose en plaques du patient suspecté, dans le cas où ladite affection est détectée.

2. Système selon la revendication 1, comprenant :
- une première source de détection (TCCS) dudit reflux sanguin dans au moins l'une des veines cérébrales profondes,
- une seconde source de détection (ECD) d'au moins l'un :
- i) dudit reflux sanguin dans au moins l'une des veines jugulaires et/ou vertébrales internes ;
- ii) de ladite sténose dans au moins l'une des veines jugulaires internes ;
- iii) de ladite absence de flux sanguin détectable au Doppler dans au moins l'une des veines jugulaires et/ou vertébrales internes ; et
- iv) dudit écart négatif entre la section transversale d'au moins l'une des veines jugulaires internes lorsque ledit patient se trouve en position allongée et en position debout, et
dans lequel ledit module de traitement est fonctionnellement raccordé auxdites première et seconde sources de détection pour détecter ladite affection.

3. Système selon la revendication 2, dans lequel ladite première source de détection comprend un appareil d'échographie duplex transcrânienne à code de couleurs (TCCS).

4. Système selon la revendication 2 ou 3, dans lequel ladite seconde source détection comprend un appareil d'échodoppler couleur extracrânien (ECD).

5. Système selon l'une quelconque des revendications précédentes, dans lequel ledit module de traitement (PC) est configuré pour attribuer des valeurs de facteur de risque respectives à l'un :
- dudit reflux sanguin dans au moins l'une des veines cérébrales profondes ;
- dudit reflux sanguin dans au moins l'une des veines jugulaires et/ou vertébrales internes ;
- de ladite sténose dans au moins l'une des veines jugulaires internes ;
- de ladite absence de flux sanguin détectable au Doppler dans au moins l'une des veines jugulaires et/ou vertébrales internes ; et
- dudit écart négatif entre les sections transversales
tels que détectés par ledit ensemble de sources de détection, et pour obtenir une valeur de facteur de risque cumulée en fonction desdites valeurs de facteur de risque respectives.

6. Système selon la revendication 5, dans lequel ledit module de traitement (PC) est configuré pour attribuer au dit reflux sanguin dans au moins l'une des veines jugulaires et/ou vertébrales internes une valeur de facteur de risque respective supérieure à toute autre valeur de facteur de risque respective attribuée par ledit module de traitement (PC).
